# EUROPEAN PATENT SPECIFICATION

(11) **EP 4 386 934 B1**
(45) Date of publication and mention of the grant of the patent: **04.02.2026**
(21) Application number: 23208672.8
(22) Date of filing: 09.11.2023
(51) Int. Cl.: H01M 10/48, H01M 50/317

(54) **SYSTEM AND METHOD FOR DETECTING THERMAL RUNAWAY USING ACOUSTIC AND GAS SENSORS**
SYSTEM UND VERFAHREN ZUR ERKENNUNG VON THERMISCHEM DURCHGEHEN MIT AKUSTISCHEN UND GASSENSOREN
SYSTÈME ET PROCÉDÉ DE DÉTECTION D'EMBALLEMENT THERMIQUE À L'AIDE DE CAPTEURS ACOUSTIQUES ET DE GAZ

(30) Priority: 12.12.2022 IN 202211071637
(43) Date of publication of application: 19.06.2024
(73) Proprietor: Honeywell International Inc., Charlotte, NC 28202 (US)
(72) Inventor: JAYAKUMAR, Prince Ashwin Kumar Anburaj, Charlotte, 28202 (US); KUMAR, Nirmal, Charlotte, 28202 (US)
(74) Representative: Haseltine Lake Kempner LLP

(56) References cited:
- EP-A1- 3 772 428
- DE-U1- 202021 106 777

## Description

### TECHNOLOGICAL FIELD

Embodiments of the present disclosure relate generally to battery management systems for detecting thermal runaway in a battery cell, and more particularly, to utilizing acoustic and gas sensors to detect the early stages of thermal runaway.

### BACKGROUND

DE 20 2021 106777 U1 mentions that an acoustic sensor can be positioned in a battery housing to receive an acoustic signal indicative of an explosion; it also mentions that a cooling compressor can be used.

Applicant has identified many technical challenges and difficulties associated with detecting the onset of thermal runaway in the early stages. Through applied effort, ingenuity, and innovation, Applicant has solved problems related to recognizing the onset of thermal runaway, which are described in detail below.

### BRIEF SUMMARY

Various embodiments are directed to example systems and methods for determining the onset of thermal runaway.

In accordance with some embodiments of the present disclosure, an example system is provided. In some embodiments, the system may comprise a battery housing defining an internal battery compartment containing a battery cell. In some embodiments, the system may further comprise an acoustic sensor positioned to receive an acoustic signal from the battery cell. In some embodiments, the system may further comprise a fan attached to the battery housing and positioned to direct a gas away from the battery cell. In some embodiments, the fan may be enabled in an instance in which the acoustic sensor detects an acoustic signal indicative of thermal runaway.

In some embodiments, the system may further comprise an internal gas sensor, wherein the internal gas sensor is positioned to receive the gas from the battery cell.

In some embodiments, the acoustic sensor, the internal gas sensor, the fan, and the battery cell may be disposed within the battery housing.

In some embodiments, the system may further comprise an external gas sensor, wherein an alert indicating thermal runaway may be transmitted in an instance in which the internal gas sensor detects an indicator gas indicative of thermal runaway and the external gas sensor does not detect the indicator gas.

In some embodiments, the system may further comprise an adaptable vent in the battery housing through which gasses in the internal battery compartment may exit.

In some embodiments, the adaptable vent may be opened when the fan is enabled and the adaptable vent may be closed when the fan is disabled.

In some embodiments, the system may further comprise a processor, wherein the processor may be communicatively connected to the acoustic sensor, the internal gas sensor, the fan, the adaptable vent, and the external gas sensor.

In some embodiments, the system may further comprise a compartment containing a mitigating substance, wherein upon detection of thermal runaway, the mitigating substance is released into the internal battery compartment.

An example method for detecting thermal runaway is further provided. In some embodiments, the method may comprise receiving acoustic data from an acoustic sensor positioned to receive an acoustic signal from a battery cell, detecting thermal runaway based at least in part on the acoustic data, and enabling a fan positioned to direct a gas away from the battery cell, based at least in part on the detection of thermal runaway.

In some embodiments, the method may further comprise receiving internal gas sensor data, from an internal gas sensor attached to a battery housing within the internal battery compartment defined by the battery housing, and detecting thermal runaway based at least in part on the internal gas sensor data.

In some embodiments, the acoustic sensor, the internal gas sensor, the fan, and the battery cell may be disposed within the battery housing.

In some embodiments, the method may further comprise receiving external gas sensor data, from an external gas sensor positioned outside the battery housing, and detecting thermal runaway based at least in part on the external gas sensor data.

In some embodiments, the method may further comprise determining that an indicator gas indicative of thermal runaway is detected by the internal gas sensor, further determining that the indicator gas is not detected by the external gas sensor; and transmitting an alert indicating thermal runaway.

In some embodiments, the method may further comprise opening an adaptable vent, disposed in a wall of the battery housing, upon detecting thermal runaway, providing fluid communication from the internal battery compartment to an environment exterior to the battery housing.

In some embodiments, upon detecting thermal runaway, the method may further comprise causing a mitigating substance to be released into the internal battery compartment.

Another example system is further provided. In some embodiments, the system may comprise an acoustic sensor positioned to receive an acoustic signal from a battery cell indicative of thermal runaway, and an internal gas sensor positioned proximate the battery cell and configured to detect an indicator gas indicative of thermal runaway. In some embodiments, in an instance in which the acoustic sensor receives the acoustic signal indicative of thermal runaway and the internal gas sensor detects the indicator gas indicative of thermal runaway, an alert indicating thermal runaway may be transmitted.

In some embodiments, the system may further comprise a battery housing defining an internal battery compartment, wherein the acoustic sensor, the internal gas sensor, and the battery cell are disposed within the internal battery compartment.

In some embodiments, the system may further comprise an external gas sensor, wherein the alert indicating thermal runaway may be transmitted in an instance in which the internal gas sensor detects the indicator gas indicative of thermal runaway and the external gas sensor does not detect the indicator gas indicative of thermal runaway.

In some embodiments, the system may further comprise an adaptable vent in the battery housing through which gasses in the internal battery compartment may pass through to the exterior of the battery housing, and wherein, the adaptable vent may be opened in an instance in which the acoustic sensor receives the acoustic signal indicative of thermal runaway.

In some embodiments, the system may further comprise a fan positioned to direct the indicator gas indicative of thermal runaway away from the battery cell, wherein, the fan may be enabled in an instance in which the acoustic sensor receives the acoustic signal indicative of thermal runaway.

### BRIEF DESCRIPTION OF THE DRAWINGS

Reference will now be made to the accompanying drawings. The components illustrated in the figures may or may not be present in certain embodiments described herein. Some embodiments may include fewer (or more) components than those shown in the figures in accordance with an example embodiment of the present disclosure.
FIG. 1 illustrates an example flowchart of the progression of thermal runaway through the various stages in accordance with an example embodiment of the present disclosure.
FIG. 2 illustrates a system-level diagram of an example thermal runaway detection system including an acoustic sensor and an internal gas sensor in accordance with an example embodiment of the present disclosure.
FIG. 3 illustrates a system-level diagram of an example thermal runaway detection system further including a fan and vent mechanism in accordance with an example embodiment of the present disclosure.
FIG. 4 illustrates a system-level diagram of an example thermal runaway detection system further including a processor in accordance with an example embodiment of the present disclosure.
FIG. 5 illustrates a system-level diagram of an example thermal runaway detection system further including a compartment containing a mitigating substance in accordance with an example embodiment of the present disclosure.
FIG. 6 illustrates an example block diagram showing example components of a processor in accordance with an example embodiment of the present disclosure.
FIG. 7 depicts a flowchart illustrating an example method for generating a thermal runaway alert in accordance with an example embodiment of the present disclosure.

### DETAILED DESCRIPTION

Example embodiments will be described more fully hereinafter with reference to the accompanying drawings, in which some, but not all embodiments of the inventions of the disclosure are shown. Indeed, embodiments of the disclosure may be embodied in many different forms and should not be construed as limited to the embodiments set forth herein; rather, these embodiments are provided so that this disclosure will satisfy applicable legal requirements. Like numbers refer to like elements throughout.

Various example embodiments address technical problems associated with determining the onset of a thermal runaway event. As understood by those of skill in the field to which the present disclosure pertains, there are numerous scenarios in which it may be advantageous to detect thermal runaway in its early stages, providing opportunity to mitigate the hazardous condition before the thermal runaway becomes irreversible.

In general, batteries (e.g., lithium-ion batteries, lithium-polymer batteries, etc.) may undergo a chemical reaction within a battery cell to supply power to various devices. Devices requiring substantial amounts of power, such as electric vehicles, may contain tens or even hundreds of battery cells in a battery pack.

Battery cells utilizing chemical reactions to generate power may be susceptible to a number of dangerous conditions. One example dangerous battery cell condition that may result from various forms of stress and/or abuse is thermal runaway. In certain circumstances, the movement of electrons and lithium ions in the battery cell may produce heat faster than the battery pack can dissipate the generated heat. Once the internal temperature of the battery cell reaches a certain point, the temperature of the battery cell may rise uncontrollably until the battery cell combusts. Not only can this dangerous condition occur in an individual battery cell but thermal runaway can cause an uncontrollable rise in temperature in the battery cells commonly contained within a battery pack, causing neighboring battery cells to also enter into thermal runaway. Eventually, a battery cell and/or battery pack, may combust causing an extremely hazardous condition. A battery cell may progress through the stages of thermal runaway rapidly, providing little time to detect and mitigate the hazardous condition before the chain reaction is irreversible.

In some instances, the stress and abuse of the battery cells resulting in thermal runaway may be immediate and apparent. For example, a battery cell on an electric vehicle may be punctured or ruptured by flying debris, leading to a thermal runaway event. In other instances, long-term abuse of a battery cell or cells over an extended period of time may also lead to thermal runaway. Overcharging, over-discharging, exposure to high and low temperatures, impacts to the battery pack, and/or physical damage to the battery cells, may all affect the internal operation of the battery cells, especially when the abuse occurs over an extended period of time. In some instances, mechanical abuse, such as a crushing force on a battery cell may lead to an internal short circuit. A short circuit may in turn increase the internal heat in the battery cell triggering a thermal runaway condition. In some instances, chronic overcharging and/or discharging may lead to plating and the formation of dendrites, which may subsequently cause short circuits. In addition, in some instances, the chemical properties of the chemicals within the battery cell may slowly change over time due to mechanical, thermal, and electrical abuses. Due to the concealed nature of many of these factors, along with the long-term effects, and sudden onset of thermal runaway, it can be difficult to predict and/or detect a thermal runaway event before it is too late.

Current battery management systems may utilize various sensors in close proximity to the battery cells to determine the condition of the battery cell based on the physical characteristics around the battery cell. For example, a temperature sensor may be placed on or near the surface of the battery cell. When the temperature measurement exceeds a pre-determined max operating temperature, the battery management system may issue a warning or alert. Similarly, a gas sensor may be placed near the battery cells configured to detect certain gases which are released when a thermal runaway event has begun. Once those gases are detected, the battery management system may issue an alert indicating the presence of a dangerous battery condition. In addition, acoustic sensors have been placed proximate the battery pack to detect changes in pressure of the battery pack.

However, in many instances, these battery management systems do not issue warnings until the battery condition (e.g., thermal runaway) has already started. For example, many of the detectable gases within a battery pack are heavy and are pulled by gravity to the lowest point of the battery pack. Depending on the placement of the gas sensor within the battery pack and the orientation of the battery pack at the time of venting gases, the vented gas may not be detected until a significant buildup has occurred and thermal runaway has progressed significantly. Similarly, temperature sensors outside of the battery cells may not detect an increase in temperature outside of the battery cells until after the interior temperature of the battery cell is already rising at an irreversible rate. Due to the chain-reaction nature of a thermal runaway event, often, once the thermal runaway event has started, it cannot be stopped. As such, in some instances the battery cell will continue to overheat and eventually combust even after the condition is detected and the warning issued. Additionally, the overheating and combustion from a single battery cell may propagate to adj acent battery cells, in some instances leading to a battery pack explosion.

Further, many battery management systems are susceptible to false alarms. For example, many battery packs allow gases to flow in and out in order to equalize the pressure of the battery pack. A gas sensor within the battery pack may detect a gas that in some instances may be indicative of a thermal runaway event. However, the gas may have entered the battery pack from the external environment and is in fact not an indicator of a thermal runaway event. Similarly, an acoustic sensor may detect extraneous sounds in or around the battery pack and falsely identify the detected extraneous sounds as an indicator of a thermal runaway event.

The various example embodiments described herein utilize various techniques to detect thermal runaway in a battery cell in the early stages while limiting false detections of thermal runaway. For example, in some embodiments, the thermal runaway detection system may include an acoustic sensor placed proximate the battery cells and positioned to capture acoustic signals emanating from the battery cells. In an instance in which the acoustic sensor detects vented gases escaping one or more battery cells in the early stages of thermal runaway, a processor communicatively connected to the acoustic sensor may determine that thermal runaway is occurring or is about to occur. In addition, a thermal runaway detection system may further utilize a fan and vent to aid in the determination of the onset of thermal runaway. For example, an adaptable vent may be placed within one or more walls of the battery pack housing. Further, a fan may be attached within the battery pack and/or within one or more walls of the battery pack housing. In some embodiments, when a thermal runaway event is detected by the acoustic sensor, the fan may be enabled and the adaptable vent opened. Enabling the fan and opening the vent may provide ventilation within the battery pack, ejecting at least some of the volatile gas from the battery pack that may further enable the thermal runaway process and eventual combustion. Enabling the fan may also promote the circulation of gases, including vented gases throughout the battery pack. In some embodiments, the circulation of gases may allow an internal gas sensor to detect gases indicative of thermal runaway at an early stage.

A thermal runaway detection system may further include an external gas sensor configured to detect gases indicative of thermal runaway outside of the battery housing. In some embodiments, the thermal runaway detection system may compare the gases detected by the internal gas sensor with gases detected by the external gas sensor. Such a comparison may allow the thermal runaway detection system to determine if the gases indicative of thermal runaway detected by the internal gas sensor are originating from within the battery pack (e.g., from the battery cells) or are flowing into the battery pack from an external source.

As a result of the herein described example embodiments and in some examples, the accuracy of a thermal runaway detection system may be greatly improved. In addition, thermal runaway may be detected in an earlier stage of progression, enabling a battery management system to mitigate the condition before the thermal runaway becomes irreversible.

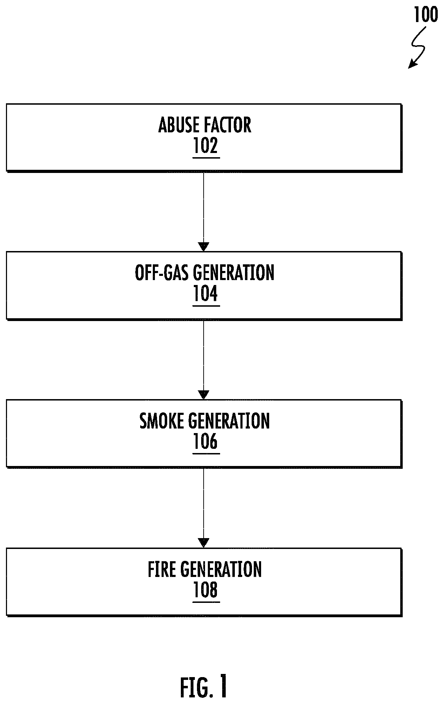

Referring now to FIG. 1, an example flowchart illustrating the stages of an example thermal runaway progression 100 in a battery cell (e.g., battery cell 202) is provided. At stage 102, an abuse factor effects one or more battery cells. A variety of abuse factors may effect the operation of a battery cell and eventually lead to thermal runaway. For example, some abuse factors may be immediate, such as physical damage to the battery cell. In such an instance, an impact may dent or crush a battery cell, in some instances causing internal shorts or exposing the internal circuitry to external influences leading to thermal runaway. In some examples, the abuse factors may be long term, or latent abuse. Such abuse factors may include chronic overcharging or discharging, as well as improper charging, or operating the battery in adverse conditions such as in high or low temperatures. Long term abuse may lead to deterioration of internal elements of the battery cell (e.g., separator or electrolyte) which subsequently lead to internal short circuits and thermal runaway.

In general, at stage 102, the battery cell is exhibiting normal operating physical characteristics. The normal operating range may vary based on the physical characteristics of the battery pack, the capacity of the battery cell, the volume of the internal battery compartment of the battery pack, the materials of the battery housing, the cooling mechanisms associated with the battery pack, and other factors. In some embodiments, the normal operating temperature, pressure, gas readings, and other physical characteristic may be determined based on normal use and/or aggregated data from users in an environment with similar environments. By way of example, the normal operating temperature may be between 20°C and 80°C and the normal operating pressure may be anything less than 14.5 pounds per square inch.

At stage 104 of the thermal runaway progression 100 off-gas generation begins. Stage 104 may be characterized by a battery cell releasing a small quantity of hydrogen, as well as volatile organic compounds. In some embodiments, the battery cell may be designed with a safety vent comprising an opening, hole, membrane, or other similar structure that facilitates the exit of any buildup of gases within the battery cell. In some embodiments, the safety vent may comprise a membrane that is intended to break and/or open when the internal pressure of the battery cell reaches a maximum internal pressure. For example, in some embodiments, the safety vent may comprise a weakened portion of the battery cell, such that when the internal pressure of the battery cell is below a maximum internal pressure, the gases are contained within the battery cell. However, if the internal pressure exceeds the pre-determined maximum internal pressure, the seal may break, opening the safety vent and facilitating the exit of any internal gases built up.

In some embodiments, the internal gases released may comprise volatile organic compounds (VOC). Volatile organic compounds are substances associated with a low boiling point at room temperature resulting in molecules that are susceptible to ready release into the surrounding air. VOCs released into the air can be highly flammable and as such add to the dangerous conditions at the onset of thermal runaway. VOCs may also be detectable by a variety of gas sensors. One of the main components of VOCs is Diethyl Carbonate (DEC). DEC is heavier than the air in the internal battery compartment of a battery pack. Once released, DEC may settle to the lowest point of the internal battery compartment, making the VOC difficult to detect depending on the placement of the gas sensors.

Stage 104 off-gas generation may be characterized by elevated levels of methane, carbon, and diethyl carbonate. In addition, stage 104 off-gas generation may be further characterized by an elevated temperature, for example, between 80°C and 100°C.

At stage 106 of the thermal runaway progression 100 smoke generation begins. Stage 104 may include the further breakdown of the electrolyte and separator layers within a battery cell. The breakdown of the separator layer, separating the anode and cathode, may result in an internal short circuit within the battery cell. Stage 106 smoke generation may be characterized by further elevated levels of VOCs such as dimethyl carbonate (DMC), diethyl carbonate (DEC), and ethyl methyl carbonate (EMC) methane, as well as further elevated levels of carbon, and carbon dioxide. In addition, visually detectable smoke may be seen in and around the battery cell. Stage 106 smoke generation may be further characterized by an elevated temperature, for example, between 100°C and 300°C and elevated pressure within the internal battery compartment. In some embodiments, once stage 106 smoke generation is reached, the onset of thermal runaway may be irreversible.

At stage 108 of the thermal runaway progression 100 fire generation begins. A battery fire is generally characterized by a temperature at or exceeding 800°C and may be accompanied by explosions. Once a battery enters the fire generation stage 108, the uncontrolled combustion is extremely hazardous to the operating device and any people and objects in close proximity.

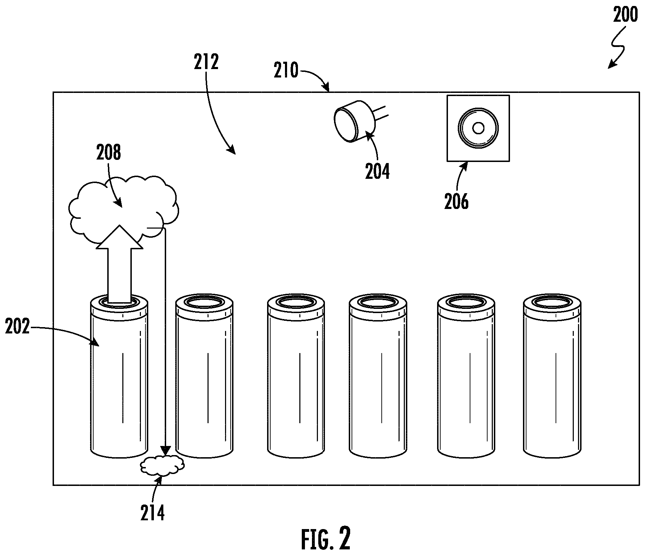

Referring now to FIG. 2, an example thermal runaway detection system 200 is provided. As depicted, the example thermal runaway detection system 200 of FIG. 2 includes a battery housing 210 defining an internal battery compartment 212 and encompassing a plurality of battery cells 202. The example thermal runaway detection system 200 further includes an acoustic sensor 204 and an internal gas sensor 206 attached to the battery housing 210 within the internal battery compartment 212. As further depicted in FIG. 2, the battery cells 202 may eject emitted gases 208. In some embodiments, the emitted gases 208 may be heavier than the air within the internal battery compartment 212 and may sink to the lowest point of the internal battery compartment 212 (e.g., heavy gases 214).

As depicted in FIG. 2, the example thermal runaway detection system 200 includes a battery housing 210 defining an internal battery compartment 212. A battery housing 210 may be any network, system, and/or compartment for enclosing and/or protecting one or more battery cells 202. In some embodiments, the battery housing may be manufactured to conform with an industry standard, such as IP67. The battery housing 210 may further define a space or compartment (e.g., internal battery compartment 212) into which the internal battery components and/or sensing devices may be disposed. In some embodiments, the battery housing 210 may comprise aluminum, steel, or other metals; plastics and/or reinforced plastics; or any other material capable of protecting the interior components and one or more battery cells 202 of the battery pack. In some embodiments, the battery housing 210 may provide structures to support, attach, and/or separate the battery cells 202, as well as wiring, and/or other internal components of the thermal runaway detection system 200. Many electric vehicles today have begun arranging large numbers of battery cells 202 into a battery housing 210 that is integrated as part of the body of the electric vehicle. This technology has largely come to be known as cell-to-chassis technology. Such a battery arrangement may be particularly prone to thermal runaway and eventual combustion as a result of mechanical, electrical and/or thermal stress and abuse.

As further depicted in FIG. 2, the thermal runaway detection system 200 includes a plurality of battery cells 202. While depicted as a plurality of battery cells 202, the disclosure is not limited to a plurality of battery cells 202 as some embodiments may include a single battery cell. The battery cells 202 may take many forms, including but not limited to cylindrical cells, prismatic cells, pouch cells, etc. The battery cells 202 may be attached and/or separated by structures defined in the battery housing 210. Additionally, the battery cells 202 may be electrically connected in parallel and/or series to provide an accumulated power output to the operating device (e.g., an electric vehicle). Each battery cell 202 may house chemical components which undergo a chemical process to generate electrical current. The chemical components of the battery cells 202 may comprise numerous compositions, for example, lithium-ion, lithium-polymer, lithium-iron phosphate, lithium Sulphur, and other lithium based compositions; nickel manganese cobalt; nickel metal hydride; lead acid; or any other chemical composition capable of providing sufficient electrical current through a chemical process. Devices requiring substantial amounts of power, such as electric vehicles, may contain tens or even hundreds of battery cells 202 encased in a battery housing 210 (e.g., battery pack). The chemical reactions occurring in these battery cells may be susceptible to dangerous battery conditions, such as thermal runaway.

As further depicted in FIG. 2, when a battery cell 202 experiences an increase in internal pressure due, for example, to a thermal runaway event, the battery cell 202 may release gases (e.g., emitted gases 208) into the surrounding environment (e.g., internal battery compartment 212). Internal pressure may build in a battery cell 202, due to a deteriorating separator layer, electrolyte degradation, increased heat due to a short circuit, and many other factors. In some embodiments, a battery cell 202 may include a safety vent to release gases (e.g., emitted gases 208) if the internal pressure of the battery cell 202 exceeds a maximum internal pressure. Emitted gases 208 may include a variety of gases depending upon the cause for venting.

Some emitted gases 208 (e.g., indicator gas) may indicate that thermal runaway is or is about to occur. An indicator gas may be any gas released into the internal battery compartment 212 during the off-gas generation stage 104 of thermal runaway as explained in relation to FIG. 1. Indicator gases may include gases emitted due to chemical reactions occurring within the battery cell 202, gases released during the degradation of a battery cell 202 component, such as the separator layer, gases resulting from the evaporation of certain substances due to heat, etc. In some examples, an indicator gas may be an abnormal or elevated level of an otherwise common gas, such as oxygen or hydrogen. In some embodiments, the indicator gas may be a VOC, including, for example, dimethyl carbonate (DMC), diethyl carbonate (DEC), and/or ethyl methyl carbonate (EMC) methane.

As further depicted in FIG. 2, some emitted gases 208 may be heavier than the air within the internal battery compartment 212 (e.g., heavy gases 214). In some embodiments, the heavy gases 214 may include molecules that cause the heavy gases 214 to sink with the pull of gravity below the air in the internal battery compartment 212. For example, the heavy gases 214 may include VOCs, such as DEC. Depending upon the orientation of the battery housing 210 and the position of one or more internal gas sensors 206, the heavy gases 214 indicating thermal runaway may be difficult to detect but a gas sensor (e.g., internal gas sensor 206). In some embodiments, the heavy gases 214 may sink to the lowest point in the direction of the pull of gravity of the internal battery compartment 212. In an instance in which the internal gas sensor 206 is positioned above the lowest point of the internal battery compartment 212 significant gas build-up may occur before the gases indicative of thermal runaway are detected. In some embodiments, the detection of thermal runaway based on measurements received by the internal gas sensor 206 may occur after thermal runaway has progressed to an irreversible point.

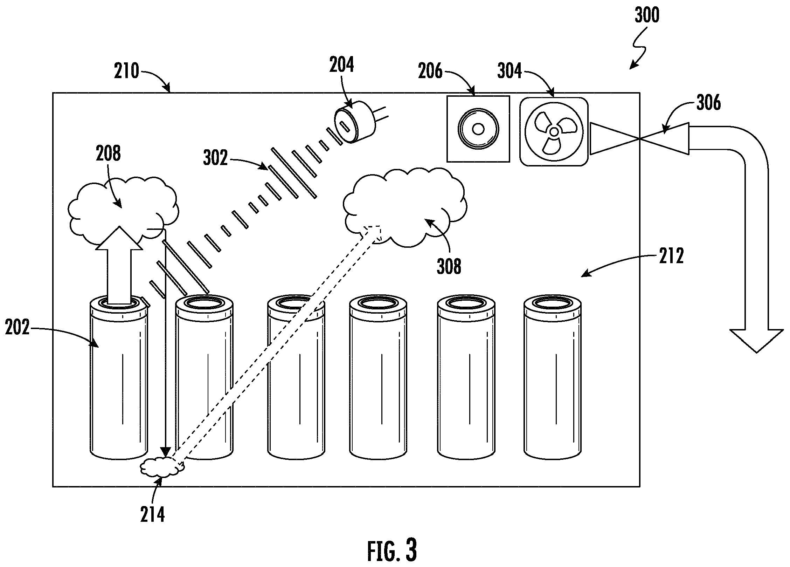

As further depicted in FIG. 2, the example thermal runaway detection system 200 further includes an acoustic sensor 204. An acoustic sensor 204 may be any electronic or electro-mechanical device capable of measuring one or more physical characteristics of an acoustic signal (e.g., acoustic signal 302 as described in connection with FIG. 3) in the surrounding environment and converting the measured one or more physical characteristics of the acoustic signal into an analog or digital electrical signal capable of being decoded. In some embodiments, an acoustic sensor 204 may measure one or more physical characteristics of an acoustic signal, such as the amplitude of the acoustic signal, the frequency of the acoustic signal, the wavelength, the time period, the velocity, or other similar physical characteristic. The detected acoustic signal in the internal battery compartment 212 may be used to determine if the battery cells 202 are experiencing venting, such as venting that occurs in the off-gas generation stage 104 of the thermal runaway progression 100. Although a single acoustic sensor 204 is depicted in the example embodiment of FIG. 2, a plurality of acoustic sensors 204 may be utilized as part of the thermal runaway detection system 200 disposed at various positions within and without the internal battery compartment 212.

As further depicted in FIG. 2, the example thermal runaway detection system 200 further includes an internal gas sensor 206. An internal gas sensor 206 may be any electronic or electro-mechanical device capable of measuring the concentration of one or more gasses in the surrounding environment and converting the measured concentration level into an analog or digital electrical signal capable of being decoded. An internal gas sensor 206 may comprise a single sensing element capable of determining the concentration level of a plurality of disparate gasses. An internal gas sensor 206 may comprise one or more sensing elements each configured to determine the concentration level of a particular gas. As non-limiting examples, the internal gas sensor 206 may determine the concentration level of methane, carbon, DEC, DMC, EMC, other VOCs, oxygen, hydrogen, and other similar gasses. The detected concentrations of these and other gases in the internal battery compartment 212 may be used to determine if gases indicative of thermal runaway (e.g., indicator gases) may be present in the internal battery compartment 212. An internal gas sensor 206, for example, may comprise but is not limited to a metal oxide gas sensor, an optical gas sensor, an electrochemical gas sensor, an acoustic gas sensor, a photoionization detector, or other similar gas sensor. Although a single internal gas sensor 206 is depicted in the example embodiment of FIG. 2, a plurality of internal gas sensors 206 may be disposed at various positions within the internal battery compartment 212 as part of the thermal runaway detection system 200.

Referring now to FIG. 3, an example thermal runaway detection system 300 is provided. As shown in FIG. 3, the example thermal runaway detection system 300 further includes a fan 304 positioned within the internal battery compartment 212. The depicted fan 304 is positioned to direct emitted gasses 308, including heavy gases 214 towards internal gas sensor 206. In addition, the example thermal runaway detection system 300 of FIG. 3 includes an adaptable vent 306 disposed in a wall of the battery housing 210 and facilitating the flow of gases (e.g., emitted gases 308) between the internal battery compartment 212 and the external environment. FIG. 3 further depicts an acoustic signal 302 emanating from the one or more battery cells 202, indicating venting of gas from the one or more battery cells 202 during the off-gas generation stage 104 of the thermal runaway progression 100.

As depicted in FIG. 3, the example thermal runaway detection system 300 includes an acoustic signal 302. An acoustic signal 302 may be any acoustic wave whether audible or inaudible with physical characteristics indicating onset of thermal runaway within an internal battery compartment 212. In some embodiments, when a battery cell 202 progresses into the off-gas generation stage 104 of thermal runaway progression 100, gases such as emitted gases 208 may be ejected and/or escape from one or more battery cells 202. The escape of such emitted gases 208 may produce an acoustic signal 302 detectable by an acoustic sensor 204. For example, the frequency and amplitude of the acoustic signal 302 may be consistent with the escape of emitted gases 208 during the off-gas generation stage 104. In some embodiments, the frequency, amplitude, wavelength, and/or other physical characteristics of the acoustic signal 302 may be indicative of off-gas generation stage 104 and thermal runaway.

As further depicted in FIG. 3, the example thermal runaway detection system 300 includes a fan 304. A fan 304 may be any apparatus, device, or other similar mechanism that may generate a flow of air in at least a portion of an internal battery compartment 212. In some embodiments, a fan 304 may be an apparatus with rotating blades which when enabled produces a current of air flow in a direction parallel to the axis of rotation of the rotating blades. In some embodiments, the fan 304 may be placed within the internal battery compartment 212. In some embodiments, the fan 304 may be disposed within one or more walls of the battery housing 210. In some embodiments, the fan 304 may be utilized to generate a current of air flow in order to ventilate the internal battery compartment 212. For example, the fan 304 may aid in clearing VOCs and other substances that may increase the likelihood of thermal runaway propagating to other battery cells 202 and/or increase the likelihood of combustion, from the internal battery compartment 212.

In some embodiments, the fan 304 may be utilized to direct the flow of VOCs and other detectable gases contained within the emitted gases 208 and indicating the onset of thermal runaway toward one or more internal gas sensors 206 (e.g., emitted gases 308). In an instance in which the emitted gases 308 are directed toward the one or more internal gas sensors 206, the detection time of thermal runaway once the off-gas generation stage 104 has commenced may be reduced. For example, in some embodiments, the indicator gases (e.g., heavy gases 214) may sink to a low point of the internal battery compartment 212, away from the internal gas sensor 206. In such an example, critical time may pass between when the emitted gases 208 are produced and when the gases reach the internal gas sensor 206. Waiting for the gases to flow to the internal gas sensor 206 organically may waste critical time in the detection of thermal runaway. In an instance in which too much time passes between the emission of gases from the battery cell 202 and detection at the internal gas sensor 206, thermal runaway may progress such that it may no longer be reversed. Thus, a fan 304 may be utilized to direct the emitted gases (e.g., emitted gases 308) toward the internal gas sensor 206. In some embodiments, the fan 304 may be placed proximate the battery cells 202 and may be directed toward the internal gas sensor 206. In some embodiments, the fan 304 may be placed proximate the internal gas sensor 206 and cause air flow such that the emitted gases 208 flow toward the internal gas sensor 206. In some examples, the fan 304 may direct a current of air flow out of the battery housing 210, for example, through a vent (e.g., adaptable vent 306), and the internal gas sensor 206 may be placed in the path of the exiting air flow.

As further depicted in FIG. 3, the example thermal runaway detection system 300 includes an adaptable vent 306. An adaptable vent 306 may be any opening in the battery housing 210 that allows the passage of air, liquid, gas, and other fluids, to and/or from the internal battery compartment 212. Although primarily described as adaptable, the adaptable vent 306 may be fixed such that the vent provides a permanent fluid connection between the internal battery compartment 212 and the environment external to the battery housing 210. In some embodiments, the adaptable vent 306 may open and close. In some embodiments, the adaptable vent 306 may be configured to receive a electrical communication causing the adaptable vent 306 to open or close. In some embodiments, the adaptable vent 306 may include an actuating element capable of opening and closing the vent. An actuating element may be any electrical, or electro-mechanical device configured to open or close the adaptable vent 306 when an electrical voltage is applied or disconnected. For example, an actuating element may be in a closed position, holding the adaptable vent 306 closed. When a voltage is applied to the actuating element, the actuating element may open, opening the adaptable vent 306. If the voltage is removed, the actuating element may return to a closed position. In some embodiments, the opening and closing of the adaptable vent 306 may be coordinated with the enabling and disabling of the fan 304. For example, in some embodiments, the adaptable vent 306 may be opened when the fan 304 is enabled, aiding the ventilation and circulation of air within the internal battery compartment 212.

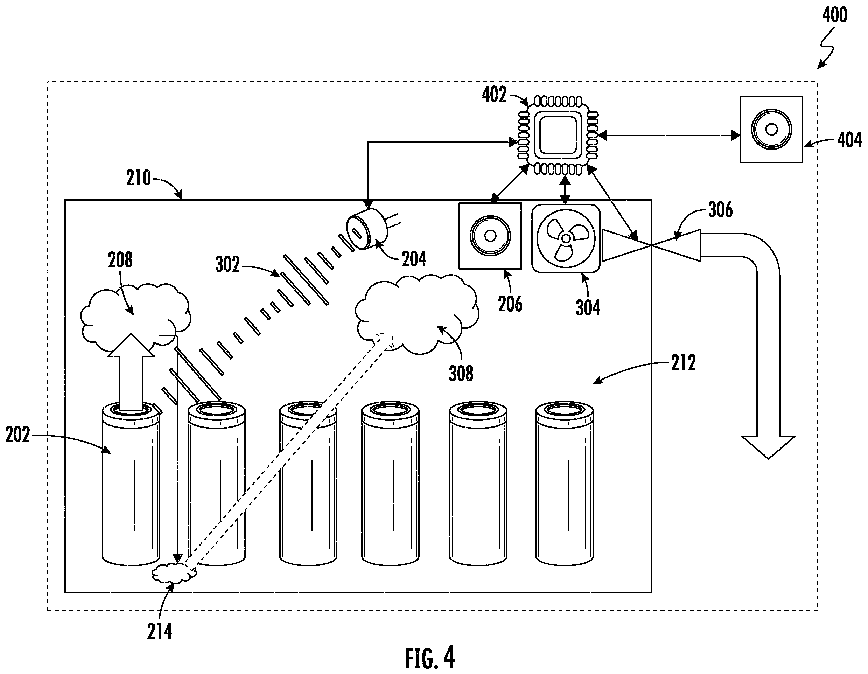

Referring now to FIG. 4, an example thermal runaway detection system 400 is provided. The example thermal runaway detection system 400 of FIG. 4 further includes a controller 402 communicatively connected to the acoustic sensor 204, the internal gas sensor 206, the fan 304, and the adaptable vent 306. Additionally, an external gas sensor 404 disposed external to the battery housing 210 is further depicted. The external gas sensor 404 is further communicatively connected to the controller 402.

As depicted in FIG. 4, the example thermal runaway detection system 400 includes an external gas sensor 404. As with the internal gas sensor 206 described in relation to FIG. 2, an external gas sensor 404 may be any electronic or electro-mechanical device capable of measuring the concentration of one or more gasses in the surrounding environment and converting the measured concentration level into an analog or digital electrical signal capable of being decoded. An external gas sensor 404 may comprise a single sensing element capable of determining the concentration level of a plurality of disparate gasses. An external gas sensor 404 may comprise one or more sensing elements each configured to determine the concentration level of a particular gas. As non-limiting examples, the external gas sensor 404 may determine the concentration level of methane, carbon, DEC, DMC, EMC, other VOCs, oxygen, hydrogen, and other similar gasses. The detected concentrations of these and other gases in the environment outside of the battery housing 210 may be used as a reference sensor. As a reference sensor, the external gas sensor 404 may aid in determining if gases indicative of thermal runaway (e.g., indicator gases) present in the internal battery compartment 212 originate from one of the battery cells 202, or originate from outside of the battery housing 210. For example, in some embodiments, many gases proximate the battery housing 210 may contain VOCs or other molecules indicative of thermal runaway. The battery housing 210 may allow external gases to enter into the internal battery compartment through the adaptable vent 306 or other similar mechanism. By also referencing the external gas sensor 404, it may be determined if the gases originate from the within the battery housing 210, an indicator of thermal runaway, or from outside the battery housing 210, indicating the detected gases are likely not indicative of thermal runaway.

An external gas sensor 404, may comprise but is not limited to a metal oxide gas sensor, an optical gas sensor, an electrochemical gas sensor, an acoustic gas sensor, a photoionization detector, or other similar gas sensor. Although a single external gas sensor 404 is depicted in the example embodiment of FIG. 4, a plurality of external gas sensors 404 may be disposed at various positions external to the battery housing 210 as part of the example thermal runaway detection system 400.

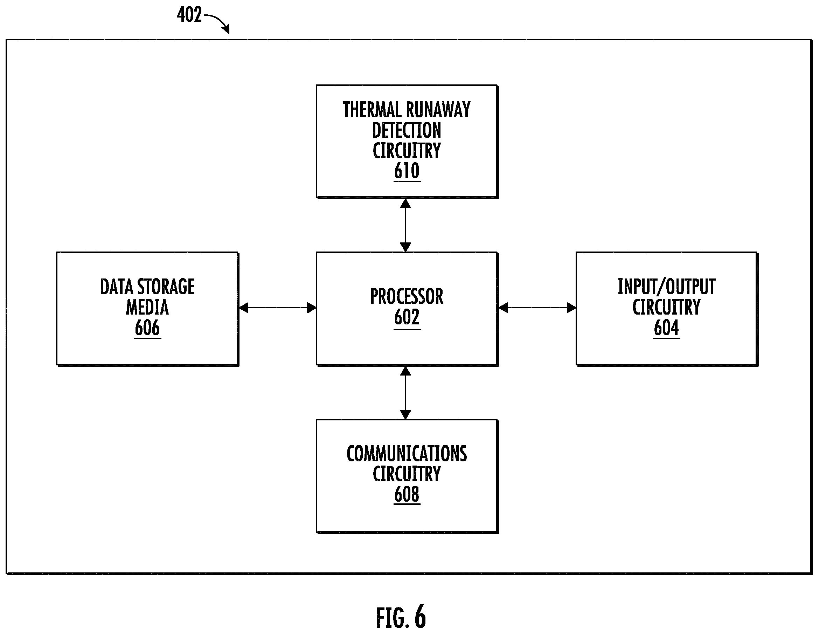

As further depicted in FIG. 4, the example thermal runaway detection system 400 includes a controller 402. A controller 402 (e.g., processor) may be any means such as a processor, device, or circuitry embodied in either hardware or a combination of hardware and software that is configured to receive input data from each of the connected sensors (e.g., one or more acoustic sensors 204, one or more internal gas sensors 206, one or more fans 304, one or more adaptable vents 306, one or more external gas sensors 404), determine the condition of the battery cells 202 based on the input data, and execute mitigating steps based on the determination. The controller 402 may comprise a form as shown and described in relation to FIG. 6. While FIG. 6 provides an example controller 402, it is noted that the scope of the present disclosure is not limited to the example shown in FIG. 6.

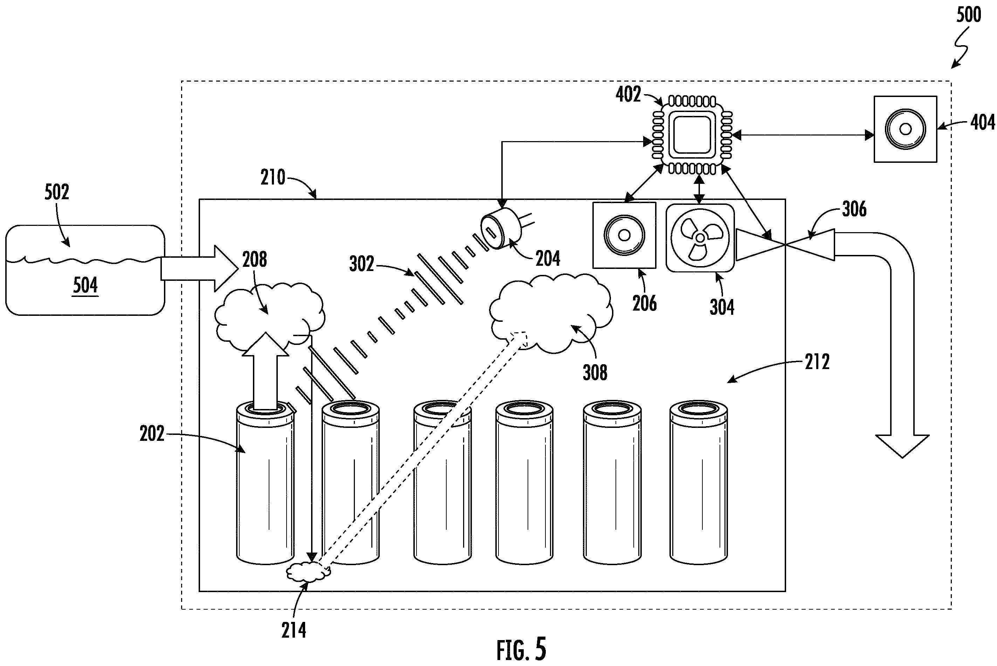

In addition, the controller 402 may be mechanically and/or electrically connected to mitigating devices, such as compartment 502 discussed in relation to FIG. 5; fan 304 and adaptable vent 306 discussed in relation to FIG. 3; as well as various communication circuitry enabling the controller 402 to communicate warnings and/or conditions related to thermal runaway. The controller 402 may utilize such mechanisms to mitigate the condition of the battery pack upon detection of thermal runaway.

Referring now to FIG. 5, an example thermal runaway detection system 500 is provided. As shown in FIG. 5, the example thermal runaway detection system 500 further includes a compartment 502 attached proximate to the battery housing 210 and fluidly connected to the internal battery compartment 212. The example compartment 502 further contains a mitigating substance 504.

As depicted in FIG. 5, the example thermal runaway detection system 500 includes a compartment 502 containing a mitigating substance 504. The compartment 502 may be any tank, reservoir, chamber, or other similar container configured to hold a mitigating substance 504. The compartment 502 may be fluidly connected to the internal battery compartment 212 such that in an instance in which thermal runaway is detected, the mitigating substance 504 may be released into the internal battery compartment 212. In some embodiments, the compartment 502 may be pressurized, such that when the mitigating substance 504 is released, the release of the contained mitigating substance 504 is accelerated.

As further depicted in FIG. 5, the compartment 502 of the example thermal runaway detection system 500 contains a mitigating substance 504. The mitigating substance 504 may be any liquid, gas, powder, solid, or other substance that may aid in inhibiting the progression of thermal runaway. For example, the mitigating substance 504 may be a powder that is released into the internal battery compartment 212 and prevents oxygen interacting with the battery cells 202, furthering the dangerous chemical reactions of thermal runaway. In some embodiments, other mitigating steps, such as disconnecting the power source and/or load from the battery cells 202 may be executed in coordination with the release of the mitigating substance into the internal battery compartment 212.

Referring now to FIG. 6, an example controller 402 in accordance with at least some example embodiments of the present disclosure is provided. The example controller 402 includes processor 602, input/output circuitry 604, data storage media 606, communications circuitry 608, and thermal runaway detection circuitry 610. In some embodiments, the controller 402 is configured, using one or more of the sets of circuitry 602, 604, 606, 608, and/or 610, to execute and perform the operations described herein.

Although components are described with respect to functional limitations, it should be understood that the particular implementations necessarily include the use of particular computing hardware. It should also be understood that in some embodiments certain of the components described herein include similar or common hardware. For example, two sets of circuitry may both leverage use of the same processor(s), network interface(s), storage medium(s), and/or the like, to perform their associated functions, such that duplicate hardware is not required for each set of circuitry. The user of the term "circuitry" as used herein with respect to components of the apparatuses described herein should therefore be understood to include particular hardware configured to perform the functions associated with the particular circuitry as described herein.

Particularly, the term "circuitry" should be understood broadly to include hardware and, in some embodiments, software for configuring the hardware. For example, in some embodiments, "circuitry" includes processing circuitry, storage media, network interfaces, input/output devices, and/or the like. Alternatively or additionally, in some embodiments, other elements of the controller 402 provide or supplement the functionality of other particular sets of circuitry. For example, the processor 602 in some embodiments provides processing functionality to any of the sets of circuitry, the data storage media 606 provides storage functionality to any of the sets of circuitry, the communications circuitry 608 provides network interface functionality to any of the sets of circuitry, and/or the like.

In some embodiments, the processor 602 (and/or co-processor or any other processing circuitry assisting or otherwise associated with the processor) is/are in communication with the data storage media 606 via a bus for passing information among components of the controller 402. In some embodiments, for example, the data storage media 606 is non-transitory and may include, for example, one or more volatile and/or non-volatile memories. In other words, for example, the data storage media 606 in some embodiments includes or embodies an electronic storage device (e.g., a computer readable storage medium). In some embodiments, the data storage media 606 is configured to store information, data, content, applications, instructions, or the like, for enabling the controller 402 to carry out various functions in accordance with example embodiments of the present disclosure.

The processor 602 may be embodied in a number of different ways. For example, in some example embodiments, the processor 602 includes one or more processing devices configured to perform independently. Additionally or alternatively, in some embodiments, the processor 602 includes one or more processor(s) configured in tandem via a bus to enable independent execution of instructions, pipelining, and/or multithreading. The use of the terms "processor" and "processing circuitry" should be understood to include a single core processor, a multi-core processor, multiple processors internal to the controller 402, and/or one or more remote or "cloud" processor(s) external to the controller 402.

In an example embodiment, the processor 602 is configured to execute instructions stored in the data storage media 606 or otherwise accessible to the processor. Alternatively or additionally, the processor 602 in some embodiments is configured to execute hard-coded functionality. As such, whether configured by hardware or software methods, or by a combination thereof, the processor 602 represents an entity (e.g., physically embodied in circuitry) capable of performing operations according to an embodiment of the present disclosure while configured accordingly. Alternatively or additionally, as another example in some example embodiments, when the processor 602 is embodied as an executor of software instructions, the instructions specifically configure the processor 602 to perform the algorithms embodied in the specific operations described herein when such instructions are executed.

As one particular example embodiment, the processor 602 is configured to perform various operations associated with utilizing various sensors and devices to detect thermal runaway in a battery pack. In some embodiments, the processor 602 includes hardware, software, firmware, and/or a combination thereof, that receives acoustic data from an acoustic sensor (e.g., acoustic sensor 204) positioned to receive an acoustic signal from a battery cell (e.g., battery cell 202). Additionally or alternatively, in some embodiments, the processor 602 includes hardware, software, firmware, and/or a combination thereof, that detects thermal runaway based at least in part on the acoustic data. Additionally or alternatively, in some embodiments, the processor 602 includes hardware, software, firmware, and/or a combination thereof, that enables a fan (e.g., fan 304) positioned to direct a gas away from the battery cell, based at least in part on the determination of the occurrence of thermal runaway. Additionally or alternatively, in some embodiments, the processor 602 includes hardware, software, firmware, and/or a combination thereof, that receives internal gas sensor data, from an internal gas sensor (e.g., internal gas sensor 206) attached to the battery housing (e.g., battery housing 210) within the internal battery compartment (e.g., internal battery compartment 212). Additionally or alternatively, in some embodiments, the processor 602 includes hardware, software, firmware, and/or a combination thereof, that detects thermal runaway based at least in part on the internal gas sensor data. Additionally or alternatively, in some embodiments, the processor 602 includes hardware, software, firmware, and/or a combination thereof, that receives external gas sensor data, from an external gas sensor (e.g., external gas sensor 404) positioned outside the battery housing. Additionally or alternatively, in some embodiments, the processor 602 includes hardware, software, firmware, and/or a combination thereof, that detects thermal runaway based at least in part on the external gas sensor data. Additionally or alternatively, in some embodiments, the processor 602 includes hardware, software, firmware, and/or a combination thereof, that determines that an indicator gas indicative of thermal runaway is detected by the internal gas sensor. Additionally or alternatively, in some embodiments, the processor 602 includes hardware, software, firmware, and/or a combination thereof, that determines that the indicator gas is not detected by the external gas sensor. Additionally or alternatively, in some embodiments, the processor 602 includes hardware, software, firmware, and/or a combination thereof, that transmits an alert indicating thermal runaway. Additionally or alternatively, in some embodiments, the processor 602 includes hardware, software, firmware, and/or a combination thereof, that opens an adaptable vent, disposed in a wall of the battery housing, upon detecting thermal runaway, providing fluid communication from the internal battery compartment to an environment exterior to the battery housing.

In some embodiments, the controller 402 includes input/output circuitry 604 that provides output to the user and, in some embodiments, to receive an indication of a user input. In some embodiments, the input/output circuitry 604 is in communication with the processor 602 to provide such functionality. The input/output circuitry 604 may comprise one or more user interface(s) (e.g., user interface) and in some embodiments includes a display that comprises the interface(s) rendered as a web user interface, an application user interface, a user device, a backend system, or the like. The processor 602 and/or input/output circuitry 604 comprising the processor may be configured to control one or more functions of one or more user interface elements through computer program instructions (e.g., software and/or firmware) stored on a memory accessible to the processor (e.g., data storage media 606, and/or the like). In some embodiments, the input/output circuitry 604 includes or utilizes a user-facing application to provide input/output functionality to a client device and/or other display associated with a user.

In some embodiments, the controller 402 includes communications circuitry 608. The communications circuitry 608 includes any means such as a device or circuitry embodied in either hardware or a combination of hardware and software that is configured to receive and/or transmit data from/to a network and/or any other device, circuitry, or module in communication with the controller 402. In this regard, the communications circuitry 608 includes, for example in some embodiments, a network interface for enabling communications with a wired or wireless communications network. Additionally or alternatively in some embodiments, the communications circuitry 608 includes one or more network interface card(s), antenna(s), bus(es), switch(es), router(s), modem(s), and supporting hardware, firmware, and/or software, or any other device suitable for enabling communications via one or more communications network(s). Additionally or alternatively, the communications circuitry 608 includes circuitry for interacting with the antenna(s) and/or other hardware or software to cause transmission of signals via the antenna(s) or to handle receipt of signals received via the antenna(s). In some embodiments, the communications circuitry 608 enables transmission to and/or receipt of data from a client device in communication with the controller 402.

The thermal runaway detection circuitry 610 includes hardware, software, firmware, and/or a combination thereof, that supports various functionality associated with configuring and/or communicating with various sensing elements, for example, one or more acoustic sensors (e.g., acoustic sensor 204), one or more internal gas sensors (e.g., internal gas sensor 206), one or more external gas sensors (e.g., external gas sensor 404), one or more fans (e.g., fan 304), one or more adaptable vents (e.g., adaptable vent 306), one or more mitigating tools (e.g., compartment 502), and/or other similar sensing elements. In some embodiments, the thermal runaway detection circuitry 610 may further include hardware, software, firmware, and/or a combination thereof, that supports various functionality associated with utilizing the received data from the various sensing elements to determine whether thermal runaway is or is about to occur in one or more of the battery cells (e.g., battery cell 202). In some embodiments, the thermal runaway detection circuitry 610 may further include hardware, software, firmware, and/or a combination thereof, that supports various functionality associated with configuring and/or communicating with mitigating tools such as compartment 502 or power switches, to reduce the likelihood of thermal runaway progressing uncontrollably when thermal runaway is first detected.

Additionally or alternatively, in some embodiments, one or more of the sets of circuitry 602-610 are combinable. Additionally or alternatively, in some embodiments, one or more of the sets of circuitry perform some or all of the functionality described associated with another component. For example, in some embodiments, one or more sets of circuitry 602-610 are combined into a single module embodied in hardware, software, firmware, and/or a combination thereof. Similarly, in some embodiments, one or more of the sets of circuitry, for example thermal runaway detection circuitry 610, is/are combined such that the processor 602 performs one or more of the operations described above with respect to each of these circuitry individually.

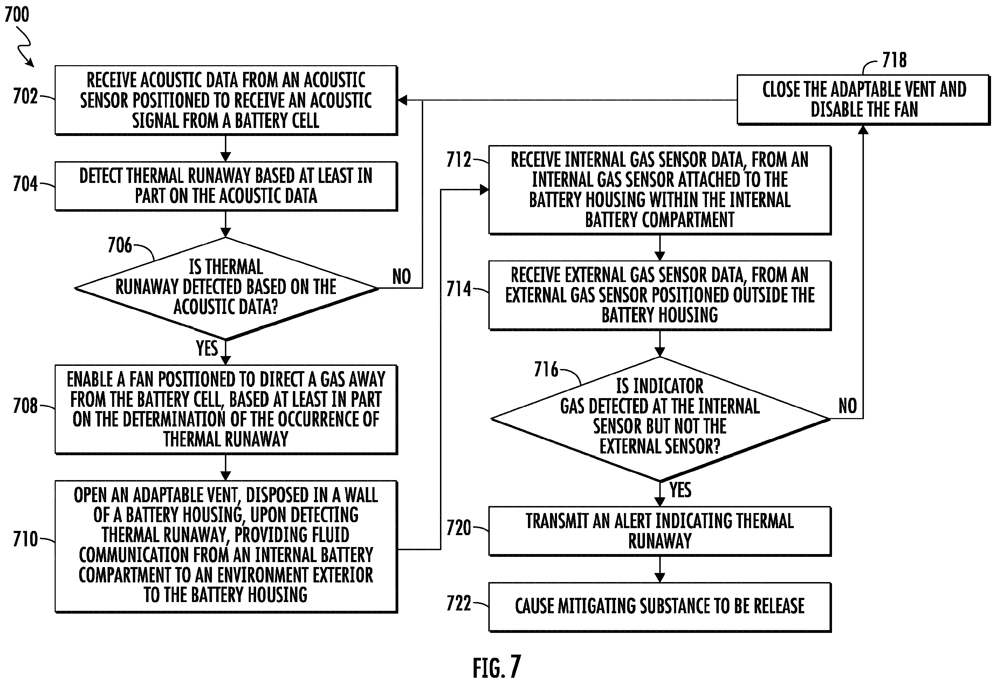

Referring now to FIG. 7, an example thermal runaway detection flowchart 700 is provided. As shown in FIG. 7, the example thermal runaway detection flowchart 700 depicts an example process for determining that thermal runaway in one or more battery cells has commenced. Although an example thermal runaway detection process is depicted by the thermal runaway detection flowchart 700, the thermal runaway detection flowchart 700 is shown for exemplary purposes and the scope of the present disclosure is not limited to the description in relation to FIG. 7.

At step 702, a controller (e.g., controller 402) receives acoustic data from an acoustic sensor (e.g., acoustic sensor 204) positioned to receive an acoustic signal (e.g., acoustic signal 302) from a battery cell (e.g., battery cell 202). In some embodiments, the acoustic sensor may be positioned proximate one or more battery cells, for example, the acoustic sensor may be attached to the battery housing within the internal battery compartment. In some embodiments, the acoustic sensor may be directional and may further be positioned such that sound emanating from the one or more battery cells is at least partially isolated. In some embodiments, the acoustic sensor may be omnidirectional and may receive acoustic signals in all directions. The acoustic sensor may convert the received acoustic signal into an electronic signal (e.g., acoustic data), and transmit the electronic signal to the controller. The controller may determine certain physical characteristics of the acoustic signal based on the acoustic data, such as the amplitude, frequency, wavelength, etc. of the received acoustic signal.

At step 704, the controller detects thermal runaway based at least in part on the acoustic data. In some embodiments, the controller may analyze the received acoustic data to determine if thermal runaway has commenced in one or more of the battery cells. The controller may perform pre-processing operations on the received acoustic data such as digital conversion, de-noising, re-sampling, smoothing, and other similar operations to prepare the acoustic data for processing. The controller may than employ a number of algorithms to determine if the received acoustic signal is indicative of the venting of gases from a battery cell during the early stages of thermal runaway. The processor may consider the amplitude, frequency, wavelength, duration, consistency, time period, the velocity, etc. to determine if the received acoustic signal is consistent with venting gases during thermal runaway. In addition, the processor may store a set duration of acoustic data to determine the change in physical properties over time. The change in physical properties of the acoustic signal over time may further be indicative of venting gases during thermal runaway. For example, the frequency of the acoustic signal may steadily decline over time and/or the amplitude of the received signal may steadily decrease over time.

In some embodiments, machine learning techniques may be used to determine if the received acoustic data is consistent with venting gas, for example, during the off-gas generation stage 104 of thermal runaway. In such an embodiment, the machine learning model may implement a supervised, semi-supervised, or unsupervised learning model to train the machine learning model to recognize the venting gases of off-gas generation based on the received acoustic data. The controller may, during operation, provide the acoustic data to the machine learning model to determine whether the acoustic data is indicative of vented gases during the off-gas generation stage 104 of thermal runaway.

At step 706, in an instance in which thermal runaway is not detected based on the acoustic data, operation returns to step 702 where the controller continues to receive acoustic data from the acoustic sensor. In an instance in which thermal runaway is detected based on the acoustic data, operation continues at step 708.

At step 708, the controller enables a fan (e.g., fan 304) positioned to direct a gas away from the battery cell, based at least in part on the determination of the occurrence of thermal runaway. Enabling a fan may provide ventilation of vented gases from the internal battery compartment. For example, in some instances, the gases vented from a battery cell may contain VOCs and other flammable substances. In an instance in which one or more battery cells are entering into thermal runaway, VOCs and other flammable gases may aid the progression of thermal runaway. Enabling a fan may direct the VOCs and other flammable gases away from the battery cells, slowing or stopping the progression of thermal runaway, especially between neighboring battery cells. In addition, enabling a fan may direct the VOCs and other flammable gases toward an internal gas sensor. In some embodiments, an internal gas sensor may be positioned in the internal battery compartment to detect the presence of VOCs and other gases indicative of thermal runaway (e.g., indicator gases). The internal gas sensor may not detect indicator gases until the indicator gases reach the internal gas sensor. The fan may direct air flow toward the internal gas sensor, speeding up the detection time of the indicator gases.

At step 710, the controller opens an adaptable vent (e.g., adaptable vent 306), disposed in a wall of a battery housing (e.g., battery housing 210), upon detecting thermal runaway, providing fluid communication from an internal battery compartment (e.g., internal battery compartment 212) to an environment exterior to the battery housing. In some embodiments, the adaptable vent may be opened in coordination with enabling the fan. The adaptable vent may provide fluid communication between the internal battery compartment and the external environment. Opening an adaptable vent may improve the ventilation of VOC and other flammable gases from within the internal battery compartment.

At step 712, the controller receives internal gas sensor data, from an internal gas sensor (e.g., internal gas sensor 206) attached to the battery housing within the internal battery compartment. Internal gas sensor data may be any data related to the concentration of certain gases detected by the internal gas sensor. In some embodiments, an internal gas sensor may be configured to detect a particular gas. The internal gas sensor data may be a signal from the internal gas sensor indicating the concentration level of the particular gas has exceeded a minimum threshold. For example, an internal gas sensor may be configured to detect DEC. In an instance in which the detected level of DEC exceeds a minimum threshold, the internal gas sensor may send a signal or message to the controller, indicating the minimum threshold has been exceeded. In some embodiments, the concentration level of one or more VOCs and/or other flammable gases may be transmitted to the controller. In such an embodiment, the controller may determine based on the concentration level of the one or more VOCs and/or other flammable gases the state of the battery cell.

At step 714, the controller receives external gas sensor data, from an external gas sensor (e.g., external gas sensor 404) positioned outside the battery housing. Similar to the internal gas sensor data, external gas sensor data may be any data related to the concentration of certain gases outside of the battery housing, as detected by the external gas sensor. In some embodiments, an external gas sensor may be configured to detect a particular gas. The external gas sensor data may be a signal from the external gas sensor indicating the concentration level of the particular gas has exceeded a minimum threshold. For example, an external gas sensor may be configured to detect DEC. In an instance in which the detected level of DEC exceeds a minimum threshold, the external gas sensor may send a signal or message to the controller, indicating the minimum threshold has been exceeded. In some embodiments, the concentration level of one or more VOCs and/or other flammable gases may be transmitted to the controller. In such an embodiment, the controller may determine based on the concentration level of the one or more VOCs and/or other flammable gases the state of the battery cell.

At step 716, the controller determines based at least in part on the acoustic signal data, the internal gas sensor data, and/or the external gas sensor data whether thermal runaway has commenced in one or more of the battery cells. In general, detection of an indicator gas at the internal gas sensor but not at the external gas sensor may indicate the indicator gases originated from within the internal battery housing and thermal runaway has begun within one or more of the battery cells. In an instance in which thermal runaway is not detected based on the gas sensor data, operation returns to step 718 where the controller continues to receive acoustic data from the acoustic sensor. In an instance in which thermal runaway is detected based on the acoustic data, operation continues at step 720.

While primarily described utilizing the acoustic sensor, the internal gas sensor, and the external gas sensor, the controller may utilize any combination of data from available sensors to determine whether thermal runaway has commenced. In some embodiments, the controller may determine based only on the acoustic data or the internal gas sensor data whether thermal runaway is underway. However, reliance on acoustic data alone, or internal gas sensor data alone, may lead to false determinations of thermal runaway, and/or slow or missed indications of thermal runaway.

In some embodiments, the controller may determine whether thermal runaway has commenced based on the acoustic data and the internal gas sensor data. Utilizing both sources of data may increase the accuracy of thermal runaway determinations. For example, in some embodiments, the battery pack may be placed in an environment with numerous noises and sounds generating acoustic signals. In some instances, the acoustic sensor may detect an acoustic signal emanating from outside the battery housing that may produce an acoustic signal mimicking a signal indicative of a thermal runaway event, such as off-gas generation. The acoustic sensor may determine that thermal runaway is in process. However, if the internal gas sensor does not detect indicator gases, the controller may determine that thermal runaway has not commenced. The reverse could also be true, meaning the internal gas sensor may detect indicator gases but there is no audible indicator detected by the acoustic sensor. The controller may utilize such data to determine that thermal runaway is in process.

In some embodiments, the controller may further utilize external gas sensor data to determine whether thermal runaway is in process in one or more of the battery cells. In some embodiments, the battery pack may be disposed in an environment containing a number of gases, including VOCs. Some of the gases and VOCs in the surrounding environment may infiltrate the internal battery compartment and may be detected by the internal gas sensor. Without an external gas sensor, the controller may conclude that the gases originated from within the battery housing and are an indicator of a thermal runaway event. However, by comparing the measurements of the internal gas sensor with the measurements of the external gas sensor, the controller may conclude that the indicator gases in the internal battery compartment originated from outside the battery housing and are not an indicator of thermal runaway within the battery housing. Utilizing internal gas sensors, acoustic sensors, and external gas sensors may improve the accuracy of the thermal runaway detection system.

At step 718, upon determination that thermal runaway is not in progress in one or more of the battery cells, the controller closes the adaptable vent and disables the fan. In some embodiments, disabling the fan while not experiencing thermal runaway may reduce the overall power consumption of the thermal runaway detection system. Further, closing the adaptable vent may protect the internal components of the battery back from dust, water, gases, and other contaminants external to the battery pack during operation.

At step 720, the controller transmits an alert indicating thermal runaway. An alert may be any indication, warning, message, auditory or visible indicator, or other similar mechanism to provide notice of the thermal runaway condition of the battery. In some embodiments, the controller (e.g., controller 402), or other connected device, may transmit a message to a battery management system or other managing device indicating the onset of thermal runaway. In such an embodiment and in some instances, the battery management system may initiate a procedure to mitigate the hazardous condition, for example, disconnecting the load/charger, releasing the mitigating substance 504 in the compartment 502, and/or initiating measures to reduce the temperature of the battery cell or cells. In some embodiments, the controller may transmit a message to a graphical user interface or another user facing mechanism alerting a user of the hazardous battery cell condition. In some embodiments, the alert may prompt user action to mitigate the thermal runaway condition. In some embodiments, a light of series of lights and/or sound or series of sounds may be utilized as an alerting mechanism for the underlying hazardous battery condition.

At step 722, the controller causes the mitigating substance to be released. In some embodiments, the controller may be communicatively connected to a compartment (e.g., compartment 502) containing a mitigating substance (e.g., mitigating substance 504). In such an embodiment, the controller may transmit a message and/or signal to unblock the path between the compartment and the internal battery compartment, allowing the flow of the mitigating substance into the internal battery compartment to slow or stop the progression of thermal runaway. In some embodiments, the compartment may be pressurized, such that when the mitigating substance is released, the release of the contained mitigating substance is accelerated.

Many modifications and other embodiments of the inventions set forth herein will come to mind to one skilled in the art to which these inventions pertain having the benefit of teachings presented in the foregoing descriptions and the associated drawings. Although the figures only show certain components of the apparatus and systems described herein, it is understood that various other components may be used in conjunction with the system. Therefore, it is to be understood that the inventions are not to be limited to the specific embodiments disclosed and that modifications and other embodiments are intended to be included within the scope of the appended claims. Moreover, the steps in the method described above may not necessarily occur in the order depicted in the accompanying diagrams, and in some cases one or more of the steps depicted may occur substantially simultaneously, or additional steps may be involved. Although specific terms are employed herein, they are used in a generic and descriptive sense only and not for purposes of limitation.

The embodiments described herein are representative only and are not intended to be limiting. Accordingly, the scope of protection is not limited by the description set out above.

Additionally, the section headings used herein are provided for consistency with the suggestions under 37 C.F.R. 1.77 or to otherwise provide organizational cues. These headings shall not limit or characterize the invention(s) set out in any claims that may issue from this disclosure.

Use of broader terms such as "comprises," "includes," and "having" should be understood to provide support for narrower terms such as "consisting of," "consisting essentially of," and "comprised substantially of" Use of the terms "optionally," "may," "might," "possibly," and the like with respect to any element of an embodiment means that the element is not required, or alternatively, the element is required, both alternatives being within the scope of the embodiment(s). Also, references to examples are merely provided for illustrative purposes, and are not intended to be exclusive.

## Claims

1. A system comprising:
a battery housing defining an internal battery compartment containing a battery cell;
an acoustic sensor positioned to receive an acoustic signal from the battery cell; and
a fan attached to the battery housing and positioned to direct a gas away from the battery cell,
wherein, the fan is enabled in an instance in which the acoustic sensor detects an acoustic signal indicative of thermal runaway.

2. The system of Claim 1, further comprising an internal gas sensor, wherein the internal gas sensor is positioned to receive the gas from the battery cell.

3. The system of Claim 2, wherein the acoustic sensor, the internal gas sensor, the fan, and the battery cell are disposed within the battery housing.

4. The system of Claim 3, further comprising an external gas sensor, wherein an alert indicating thermal runaway is transmitted in an instance in which the internal gas sensor detects an indicator gas indicative of thermal runaway and the external gas sensor does not detect the indicator gas.

5. The system of Claim 4, further comprising an adaptable vent in the battery housing through which gasses in the internal battery compartment exit.

6. The system of Claim 5, wherein the adaptable vent is opened when the fan is enabled and the adaptable vent is closed when the fan is disabled.

7. The system of Claim 5, further comprising a processor, wherein the processor is communicatively connected to the acoustic sensor, the internal gas sensor, the fan, the adaptable vent, and the external gas sensor.

8. The system of Claim 4, further comprising a compartment containing a mitigating substance, wherein upon detection of thermal runaway, the mitigating substance is released into the internal battery compartment.

9. A method for detecting thermal runaway, the method comprising:
receiving acoustic data from an acoustic sensor positioned to receive an acoustic signal from a battery cell;
detecting thermal runaway based at least in part on the acoustic data; and
enabling a fan positioned to direct a gas away from the battery cell, based at least in part on the detection of thermal runaway.

10. The method of Claim 9, further comprising:
receiving internal gas sensor data, from an internal gas sensor attached to a battery housing within the internal battery compartment defined by the battery housing; and
detecting thermal runaway based at least in part on the internal gas sensor data.

11. The method of Claim 10, wherein the acoustic sensor, the internal gas sensor, the fan, and the battery cell are disposed within the battery housing.

12. The method of Claim 10, further comprising:
receiving external gas sensor data, from an external gas sensor positioned outside the battery housing; and
detecting thermal runaway based at least in part on the external gas sensor data.

13. The method of Claim 12, further comprising:
determining that an indicator gas indicative of thermal runaway is detected by the internal gas sensor;
determining that the indicator gas is not detected by the external gas sensor; and
transmitting an alert indicating thermal runaway.

14. The method of Claim 10, further comprising:
opening an adaptable vent, disposed in a wall of the battery housing, upon detecting thermal runaway, providing fluid communication from the internal battery compartment to an environment exterior to the battery housing.

15. The method of Claim 11, further comprising upon detecting thermal runaway, causing a mitigating substance to be released into the internal battery compartment.

## Patentansprüche

1. System, umfassend:
ein Batteriegehäuse, das ein Batterieinnenfach definiert, das eine Batteriezelle enthält;
einen Akustiksensor, der dazu positioniert ist, ein Akustiksignal von der Batteriezelle zu empfangen; und
einen Lüfter, der an dem Batteriegehäuse angebracht und dazu positioniert ist, ein Gas von der Batteriezelle weg zu lenken,
wobei der Lüfter in einem Fall aktiviert wird, in dem der Akustiksensor ein Akustiksignal erkennt, das anzeigend für ein thermisches Durchgehen ist.

2. System nach Anspruch 1, ferner umfassend einen Gasinnensensor, wobei der Gasinnensensor dazu positioniert ist, das Gas von der Batteriezelle zu empfangen.

3. System nach Anspruch 2, wobei der Akustiksensor, der Gasinnensensor, der Lüfter und die Batteriezelle innerhalb des Batteriegehäuses angeordnet sind.

4. System nach Anspruch 3, ferner umfassend einen Gasaußensensor, wobei ein Alarm, der ein thermisches Durchgehen anzeigt, in einem Fall gesendet wird, in dem der Gasinnensensor ein Indikatorgas erkennt, das anzeigend für thermisches Durchgehen ist, und der Gasaußensensor das Indikatorgas nicht erkennt.

5. System nach Anspruch 4, ferner umfassend eine anpassbare Belüftungsöffnung in dem Batteriegehäuse, durch die Gase in dem Batterieinnenfach austreten.

6. System nach Anspruch 5, wobei die anpassbare Belüftungsöffnung geöffnet ist, wenn der Lüfter aktiviert ist und die anpassbare Belüftungsöffnung geschlossen ist, wenn der Lüfter deaktiviert ist.

7. System nach Anspruch 5, ferner umfassend einen Prozessor, wobei der Prozessor kommunikativ mit dem Akustiksensor, dem Gasinnensensor, dem Lüfter, der anpassbaren Belüftungsöffnung und dem Gasaußensensor verbunden ist.

8. System nach Anspruch 4, ferner umfassend ein Fach, das eine Begrenzungssubstanz enthält, wobei bei Erkennen des thermischen Durchgehens die Begrenzungssubstanz in das Batterieinnenfach abgegeben wird.

9. Verfahren zum Erkennen von thermischem Durchgehen, das Verfahren umfassend:
Empfangen von Akustikdaten von einem Akustiksensor, der dazu positioniert ist, ein Akustiksignal von einer Batteriezelle zu empfangen;
Erkennen von thermischem Durchgehen auf Basis mindestens teilweise der Akustikdaten; und
Aktivieren eines Lüfters, der dazu positioniert ist, ein Gas von der Batteriezelle weg zu lenken, auf Basis mindestens teilweise der Erkennung von thermischem Durchgehen.

10. Verfahren nach Anspruch 9, ferner umfassend:
Empfangen von Gasinnensensordaten von einem Gasinnensensor, der an einem Batteriegehäuse innerhalb des von dem Batteriegehäuse definierten Batterieinnenfachs angebracht ist; und
Erkennen von thermischem Durchgehen auf Basis mindestens teilweise der Gasinnensensordaten.

11. Verfahren nach Anspruch 10, wobei der Akustiksensor, der Gasinnensensor, der Lüfter und die Batteriezelle innerhalb des Batteriegehäuses angeordnet sind.

12. Verfahren nach Anspruch 10, ferner umfassend:
Empfangen von Gasaußensensordaten von einem Gasaußensensor, der außerhalb des Batteriegehäuses positioniert ist; und
Erkennen von thermischem Durchgehen auf Basis mindestens teilweise der Gasaußensensordaten.

13. Verfahren nach Anspruch 12, ferner umfassend:
Bestimmen, dass ein Indikatorgas, das anzeigend für thermisches Durchgehen ist, von dem Gasinnensensor erkannt wird;
Bestimmen, dass das Indikatorgas nicht von dem Gasaußensensor erkannt wird; und
Senden eines Alarms, der thermisches Durchgehen anzeigt.

14. Verfahren nach Anspruch 10, ferner umfassend:
Öffnen einer anpassbaren Belüftungsöffnung, die in einer Wand des Batteriegehäuses angeordnet ist, bei Erkennen von thermischem Durchgehen, Bereitstellen einer Fluidkommunikation von dem Batterieinnenfach zu einer Umgebung außerhalb des Batteriegehäuses.

15. Verfahren nach Anspruch 11, ferner umfassend bei Erkennen von thermischem Durchgehen, Veranlassen, dass eine Begrenzungssubstanz in das Batterieinnenfach abgegeben wird.

## Revendications

1. Système comprenant :
un logement de batterie définissant un compartiment de batterie interne contenant un élément de batterie ;
un capteur acoustique positionné pour recevoir un signal acoustique provenant de l'élément de batterie ; et
un ventilateur fixé au logement de batterie et positionné pour éloigner un gaz de l'élément de batterie,
dans lequel le ventilateur est activé dans un cas où le capteur acoustique détecte un signal acoustique indiquant un emballement thermique.

2. Système selon la revendication 1, comprenant en outre un capteur de gaz interne, dans lequel le capteur de gaz interne est positionné pour recevoir le gaz provenant de l'élément de batterie.

3. Système selon la revendication 2, dans lequel le capteur acoustique, le capteur de gaz interne, le ventilateur et l'élément de batterie sont disposés à l'intérieur du logement de batterie.

4. Système selon la revendication 3, comprenant en outre un capteur de gaz externe, dans lequel une alerte indiquant un emballement thermique est transmise dans un cas où le capteur de gaz interne détecte un gaz indicateur indiquant un emballement thermique et où le capteur de gaz externe ne détecte pas le gaz indicateur.

5. Système selon la revendication 4, comprenant en outre un évent adaptable dans le logement de batterie par lequel les gaz dans le compartiment de batterie interne sortent.

6. Système selon la revendication 5, dans lequel l'évent adaptable est ouvert lorsque le ventilateur est activé et l'évent adaptable est fermé lorsque le ventilateur est désactivé.

7. Système selon la revendication 5, comprenant en outre un processeur, dans lequel le processeur est connecté de manière communicative au capteur acoustique, au capteur de gaz interne, au ventilateur, à l'évent adaptable et au capteur de gaz externe.

8. Système selon la revendication 4, comprenant en outre un compartiment contenant une substance d'atténuation, dans lequel lors de la détection d'un emballement thermique, la substance d'atténuation est libérée dans le compartiment de batterie interne.

9. Procédé de détection d'emballement thermique, le procédé comprenant :
la réception de données acoustiques provenant d'un capteur acoustique positionné pour recevoir un signal acoustique provenant d'un élément de batterie ;
la détection d'un emballement thermique sur la base au moins en partie des données acoustiques ; et
l'activation d'un ventilateur positionné pour éloigner un gaz de l'élément de batterie, sur la base au moins en partie de la détection d'un emballement thermique.

10. Procédé selon la revendication 9, comprenant en outre :
la réception de données de capteur de gaz interne, provenant d'un capteur de gaz interne fixé à un logement de batterie à l'intérieur du compartiment de batterie interne défini par le logement de batterie ; et
la détection d'un emballement thermique sur la base au moins en partie des données de capteur de gaz interne.

11. Procédé selon la revendication 10, dans lequel le capteur acoustique, le capteur de gaz interne, le ventilateur et l'élément de batterie sont disposés à l'intérieur du logement de batterie.

12. Procédé selon la revendication 10, comprenant en outre :
la réception de données de capteur de gaz externe, provenant d'un capteur de gaz externe positionné à l'extérieur du logement de batterie ; et
la détection d'un emballement thermique sur la base au moins en partie des données de capteur de gaz externe.

13. Procédé selon la revendication 12, comprenant en outre :
le fait de déterminer qu'un gaz indicateur indiquant un emballement thermique est détecté par le capteur de gaz interne ;
le fait de déterminer que le gaz indicateur n'est pas détecté par le capteur de gaz externe ; et
la transmission d'une alerte indiquant un emballement thermique.

14. Procédé selon la revendication 10, comprenant en outre :
l'ouverture d'un évent adaptable, disposé dans une paroi du logement de batterie, lors de la détection d'un emballement thermique, assurant une communication fluidique du compartiment de batterie interne vers un environnement extérieur au logement de batterie.

15. Procédé selon la revendication 11, comprenant en outre, lors de la détection d'un emballement thermique, la libération d'une substance d'atténuation dans le compartiment de batterie interne.
